# EUROPEAN PATENT APPLICATION

(11) **EP 2 302 065 A1**
(43) Date of publication of application: **30.03.2011**
(21) Application number: 10176029.6
(22) Date of filing: 19.01.2001
(51) Int. Cl.: C12P 7/64

(54) **Solventless extraction process**

(30) Priority: 19.01.2000 US 177125 P
(62) Divisional of application: 01942672.5
(71) Applicant: MARTEK BIOSCIENCES CORPORATION, Columbia, MD 21045 (US)
(72) Inventor: Ruecker, Craig M., San Diego, CA 92129 (US); Adu-Peasah, Swithin Patrick, San Diego, CA 92131 (US); Engelhardt, Brian S., San Diego, CA 92117 (US); Veeder, George T III, Ramona, CA 92065 (US)
(74) Representative: Mallalieu, Catherine Louise

(57) **Abstract**

The present invention provides a method for extracting lipids from microorganisms without using a nonpolar organic solvent as an extraction solvent. In particular, the present invention provides a method for extracting lipids from microorganisms by lysing cells and removing water soluble compound and/or materials by washing the lysed cell mixtures with aqueous washing solutions until a substantially non-emulsified lipid is obtained.

## Description

### FIELD OF THE INVENTION

The present invention is directed to a process for extracting lipids from microorganisms without the use of any significant amount of a nonpolar organic solvent.

### BACKGROUND OF THE INVENTION

A typical microorganism lipid manufacturing process, such as production of omega-3 highly unsaturated fatty acid, in particular, a lipid mixture rich in docosahexaenoic acid (DHA), involves growing microorganisms which are capable of producing the desired lipid in a fermentor, pond or bioreactor, isolating the microbial biomass, drying it, and extracting intracellular lipids with a nonpolar organic solvent, *e.g*., hexane. Generally, intracellular lipids of microorganisms are extracted after rupturing (i.e., lysing) dried cells of the microorganisms. The extracted lipids are can be further refined to produce high purity and/or quality lipids. The microorganisms are generally isolated by first diluting the fermentation broth with water, and centrifuging the mixture to isolate microorganisms. The cells are then dried and if the lipids are not extracted immediately or soon thereafter, the cells are packaged, for example, in vacuum-sealed bags, to prevent degradation of lipids.

Unfortunately, the drying process exposes the microorganisms to heat, which can damage, *i.e*., degrade the quality of, lipids if done incorrectly. The vacuum-sealed bags may develop leaks, which can further degrade the quality of the lipids due to exposure of the microorganisms to air. In addition, if the dried microorganisms are not treated with an antioxidant, lipids can be further degraded due to exposure to air and or light. For example, carotenoids, xanthophylls and long chain fatty acids like DHA may degrade due to oxidation by air and/or light. Furthermore, in some cases operators who are exposed to the dried microorganisms can develop an allergic reaction creating a safety and/or health hazard to operators.

Moreover, in an industrial scale production, the large amount of volatile and flammable nonpolar organic solvent used in lipid extraction can create hazardous operating conditions. The use of nonpolar organic solvent in the extraction process may necessitate using an explosion-proof oil recovery system, thereby adding to the cost of lipid recovery. Moreover, use of an nonpolar organic solvent in extracting lipids from microorganisms generates an nonpolar organic solvent waste stream requiring a proper disposal method, which further increases the overall production cost of lipid extraction.

Therefore, there is a need for a process for extracting lipids from microorganisms which does not require the use of a nonpolar organic solvent. There is also a need for a lipid extraction process from microorganisms which does not require the expensive step of drying the microorganisms prior to extraction.

### SUMMARY OF THE INVENTION

One embodiment of the present invention provides a process for obtaining lipid from microorganisms comprising:
(a) lysing cells of the microorganisms to produce a lysed cell mixture;
(b) treating the lysed cell mixture to produce a phase separated mixture comprising a heavy layer and a lipid-rich light layer;
(c) separating the heavy layer from the lipid-rich light layer; and
(d) obtaining the lipid and/or lipid fractions from the light layer.

Another embodiment of the present invention provides a process for obtaining lipids for microorganisms comprising:
(a) growing microorganisms in a culture medium;
(b) treating said culture medium and the microorganism cells to release intercellular lipids;
(c) subjecting the culture medium containing the released intercellular lipids to gravity separation to form a light lipid-containing phase and a heavy phase;
(d) separating said light phase from said heavy phase;
(e) treating said light phase to break an emulsion formed between said lipid and water; and
(f) recovering a crude lipid.

In accordance with another embodiment of the present invention, a process is provided for recovering lipids from microorganisms comprising the steps:
(a) growing said microorganisms in a culture medium;
(b) treating microorganism cells from said culture medium without drying said cells to release intercellular lipids;
(c) subjecting the culture medium containing the released intercellular lipids to gravity separation to form a light lipid-containing phase and a heavy phase;
(d) separating said light phase from said heavy phase;
(e) treating said light phase to break an emulsion formed between said lipid and water; and
(f) recovering a crude lipid.

Preferably, the microorganisms are cultured in a fermentation medium in a fermentor. Alternatively, the microorganisms can be cultured photosynthetically in a photobioreactor or pond. Preferably, the microorganisms are lipid-rich microorganisms, more preferably, the microorganisms are selected from the group consisting of algae, bacteria, fungi and protists, more preferably, the microorganisms are selected from the group consisting of golden algae, green algae, dinoflagelates, yeast, fungi of the genus *Mortierella,* and Stramenopiles. Preferably, the microorganisms comprise microorganisms of the genus Mortierella, genus Crypthecodinium, and order Thraustochytriales, and more preferably, microorganisms are selected from the genus *Thraustochytrium, Schizochytrium* or mixtures thereof, more preferably, the microorganisms are selected from the group consisting of microorganisms having the identifying characteristics of ATCC number 20888, ATCC number 20889, ATCC number 20890, ATCC number 20891 and ATCC number 20892, strains of *Mortierella schmuckerii,* strains of *Crypthecodinium cohnii,* mutant strains derived from any of the foregoing, and mixtures thereof.

The treatment of the cells includes a treatment to release the lipids such as lysing, rupturing or permeabilizing. As used herein, the terms lyse, lysing, lysed, etc., will be used generically to refer to a treatment to release intercellular lipids, including breaking or permeabilizing the cells. Preferably, the treatment is selected from the group consisting of heating the cells, exposing the cells to basic conditions, exposing the cells to a chelating compound or combinations thereof. More preferably the lysing or rupturing of the cells comprises heating the cells to at least 50°C while exposing the cells to basic conditions, a chelating compound or mixtures thereof.

Preferably, the gravity separation comprises passing the fermentation broth containing the released intercellular lipids through a centrifuge, such as stacked-disc-, separator- or decanter-type centrifuges.

The separated lysed cell mixture comprises a heavy layer which comprises an aqueous solution which contains the solid materials resulting from the lysed cells, and a light layer which contains lipids. The light and heavy layers can be separated by centrifugation. The lipids may be present in an emulsified state. The light layer can be further washed with an aqueous washing solution until the lipid becomes substantially non-emulsified. Preferably the treatment to break the emulsion comprises mixing the emulsion with water, alcohol, acetone or mixtures thereof and subjecting the mixture to gravity separation. Preferably, the process is performed without using nonpolar organic solvents such as hexane.

When the lipid extraction process of the present invention includes using microorganisms from a fermentation process, the extraction process can also include solubilizing at least part of proteinaceous compounds in a fermentation broth by adding a base selected from the group consisting of hydroxides, carbonates, bicarbonates, phosphates and mixtures thereof.

The process of the present invention can also include heating the microorganisms to temperature of at least about 50 °C. Preferably, a chemical compound, such as a base, is added to the culture medium to aid in the lysing of the cells.

As an alternative to heating, the cells can be lysed with the assistance of a chelating compound such as EDTA. In addition to assisting in lysing or rupturing the cells, the chelators assist in preventing oxidation of the lipids during processing by chelating (binding with) free radical-producing metal ions in the fermentation broth such as iron or copper. Preferred forms of chelators are those that are considered food grade or GRAS (Generally Recognized As Safe). Effective chelating compounds include EDTA, citric acid or citrate, lactic acid, trisodium phosphate, polyphosphate, hexametaphosphate, EGTA, DTPA, phytic acid, or CDTA and other salt forms of these compounds. In one embodiment, sodium EDTA is added to the cells to degrade the cell walls by chelating the divalent cations which assist in holding the cell walls together. The process can be performed at higher temperatures with less EDTA or lower temperatures with higher concentrations of EDTA. For example, we have found that DHA-rich cells of *Schizochytrium* sp. can be permeabilized and/or ruptured by addition of EDTA to the cultures at the end of the fermentation process. A concentration of 10,000 ppm is required to assist in rupturing the cells at 30°C, a concentration of 5,000 ppm at 50°C and at temperatures above 70°C concentrations of lower than 1000 ppm are effective. Chelators can be added to the fermentation broth to make the cells easier to break by physical processes such as homogenization. In addition to a chelator, water can also be added to increase the internal osmotic pressure in order to lyse the cells.

Preferably, the microorganisms are capable of growth at salinity level of less than about 12 g/L of sodium chloride, more preferably less than about 5 g/L of sodium chloride and most preferably less than about 3 g/L of sodium chloride. Preferably, the microorganisms are capable of growth at salinity levels of less than about 7 g/L of sodium and less than about 250 mg/L of chloride. Preferably, the chloride is present in an amount from about 70 to about 150 mg/L.

Preferably, the microorganisms comprise at least about 20% by weight of lipid, more preferably at least about 30% by weight, and most preferably at least about 40%. Alternatively at least about 20%, more preferably, at least about 20% of the lipid is cholesterol, phytosterols, desmosterol, tocotrienols, tocopherols, ubiquinones, carotenoids and xanthophylls such as beta-carotene, lutein, lycopene, astaxanthin, zeaxanthin, canthaxanthin, and fatty acids such as conjugated linoleic acids, and omega-3 and omega-6 highly unsaturated fatty acids such as eicosapentaenoic acid, docosapentaenoic acid, and docosahexaenoic acid, arachidonic acid, stearidonic acid, dihomogammalinolenic acid and gamma-linolenic acid or mixtures thereof, preferably at least about 30%, and more preferably at least about 40%.

In one particular aspect of the present invention the microorganisms are capable of producing at least about 0.1 grams per liter per hour of a mixture of lipids, preferably including cholesterol, phytosterols, desmosterol, tocotrienols, tocopherols, ubiquinones, carotenoids and xanthophylls such as beta-carotene, lutein, lycopene, astaxanthin, zeaxanthin, canthaxanthin, and fatty acids such as conjugated linoleic acids, and omega-3 and omega-6 highly unsaturated fatty acids such as eicosapentaenoic acid, docosapentaenoic acid, and docosahexaenoic acid, arachidonic acid, stearidonic acid, dihomogammalinolenic acid and gamma-linolenic acid or mixtures thereof, more preferably at least about 0.2 g/L/h, still more preferably at least about 0.3 g/L/h, and most preferably at least about 0.4 g/L/h.

In another aspect of the present invention, the microorganism is selected from the group consisting of algae, fungi, bacteria and protists. Preferably, the microorganisms are of the order Thraustochytriales. More preferably the microorganisms are selected from the genus *Thraustochytrium, Schizochytrium* and mixtures thereof. And most preferably, the microorganisms are selected from the group consisting of microorganisms having the identifying characteristics of ATCC number 20888, ATCC number 20889, ATCC number 20890, ATCC number 20891 and ATCC number 20892, mutant strains derived from any of the foregoing, and mixtures thereof. Preferably, the microorganisms are selected from the group consisting of microorganisms having the identifying characteristics of ATCC number 20888 and ATCC number 20889, and more preferably ATCC number 20888, mutant strains derived from any of the foregoing, and mixtures thereof.

### BRIEF DESCRIPTION OF THE DRAWINGS

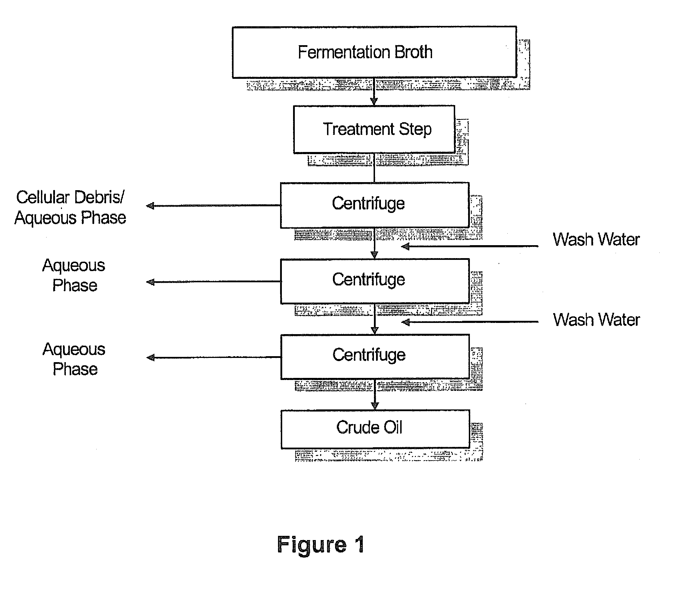

Figure 1 is a flow diagram of one embodiment of a solventless extraction process of the present invention.

### DETAILED DESCRIPTION OF THE INVENTION

The present invention is directed to a process for extracting, recovering, isolating or obtaining lipids from microorganisms. The process of the present invention is applicable to extracting a variety of lipids from a variety of microorganisms, for example, extracting lipids containing cholesterol, phytosterols, desmosterol, tocotrienols, tocopherols, ubiquinones, carotenoids and xanthophylls such as beta-carotene, lutein, lycopene, astaxanthin, zeaxanthin, canthaxanthin, and fatty acids such as conjugated linoleic acids, and omega-3 and omega-6 highly unsaturated fatty acids such as eicosapentaenoic acid, docosapentaenoic acid, and docosahexaenoic acid, arachidonic acid, stearidonic acid, dihomogammalinolenic acid and gamma-linolenic acid or mixtures thereof, more preferably, omega-3 highly unsaturated fatty acids, such as docosahexaenoic acid (DHA), eicosapentaenoic acid (EPA), and/or docosapentaenoic acid (DPA) (i.e., the omega-3 form of DPA), in particular lipids containing a relatively large amount of DHA, from microorganisms producing the same; and extracting lipids containing omega-6 highly unsaturated fatty acids, such as arachidonic acid and docosapentaenoic acid (DPA) (i.e., the omega-6 form of DPA)from microorganisms producing the same. Exemplary microorganisms which produce a relatively large amount of omega-3 highly unsaturated fatty acids are disclosed in commonly assigned U.S. Patent Nos. 5,340,594 and 5,340,742, both issued to Barclay, and exemplary microorganisms which produce a relatively large amount of arachidonic acid are disclosed in commonly assigned U.S. Patent No. 5,583,019, issued to Barclay. All of the above disclosed patents are incorporated herein by reference in their entirety.

For the sake of brevity, however, this detailed description of the invention is presented for purposes of convenience and illustration for the case of extracting lipids comprising omega-3 highly unsaturated fatty acid from microorganisms producing the same, in particular extracting lipids from microorganisms that produce a relatively high amount of DHA. It is to be understood, however, that the invention as a whole is not intended to be so limited, and that one skilled in the art will recognize that the concept of the present invention will be applicable to other microorganisms producing a variety of lipid compositions in accordance with the techniques discussed herein. These microorganisms include microorganisms, such as fungi, protists, algae and bacteria, which produce a variety of lipids, such as phospholipids; free fatty acids; esters of fatty acids, including triglycerides of fatty acids; sterols; pigments (*e.g*., carotenoids and oxycarotenoids) and other lipids, and lipid associated compounds such as phytosterols, ergothionine, lipoic acid and antioxidants including beta-carotene, tocotrienols, and tocopherol. Preferred lipids and lipid associated compounds include, but are not limited to, cholesterol, phytosterols, desmosterol, tocotrienols, tocopherols, ubiquinones, carotenoids and xanthophylls such as beta-carotene, lutein, lycopene, astaxanthin, zeaxanthin, canthaxanthin, and fatty acids such as conjugated linoleic acids, and omega-3 and omega-6 highly unsaturated fatty acids such as eicosapentaenoic acid, docosapentaenoic acid, and docosahexaenoic acid, arachidonic acid, stearidonic acid, dihomogammalinolenic acid and gamma-linolenic acid or mixtures thereof. For the sake of brevity, unless otherwise stated, the term "lipid" refers to lipid and/or lipid associated compounds. Other lipids and microorganisms which may be suitable for use in the instant invention will be readily apparent to those skilled in the art.

Typical microbial lipid (in particular an oil containing an omega-3 highly unsaturated fatty acid such as DHA) manufacturing processes involve growing microorganisms which produce DHA in a fermentor, isolating the microorganisms, drying the microbial biomass and extracting the intracellular lipids with an non-polar organic solvent, *e.g*., hexane. The extracted lipid is generally further refined to produce a high purity and/or quality lipid. The isolation of microorganisms involves diluting the fermentation broth with water and centrifuging the mixture to isolate microorganisms. When lipids are not extracted immediately or soon after isolating the microorganisms, the isolated microorganisms are typically dried, *for example,* on a drum dryer, and sealed in a package, *e.g*., in vacuum-sealed bags, to prevent degradation of lipids. Unfortunately, the drying process exposes the microorganisms to heat, which can damage, *i.e.*, degrade the quality of, the lipid if done incorrectly. The package may develop leaks, which can further degrade the quality of the lipids. Furthermore, if the dried microorganisms are not treated with an antioxidant, the exposure of microorganisms to air and/or light can further degrade the lipids.

Recovering the crude oil directly from the fermentation broth avoids these problems. Avoiding the non-polar organic solvent extraction step reduces manufacturing costs and also eliminates operator exposure to the dried microorganisms, which can cause an allergic response in some individuals.

The present invention provides a method for obtaining lipids from microorganisms using a substantially nonpolar organic solvent free extraction process, *i.e.* a "solventless" extraction process. The term "solventless extraction process" refers to an extraction process which, when an aqueous or polar solvent is used, the aqueous or polar solvent includes less than about 5% of a nonpolar organic solvent, preferably less than about 4%, more preferably less than about 2%, and most preferably less than 1%. However, solvents can be employed in downstream steps such as in a refining process. The process of the present invention can include obtaining or isolating microorganisms, preferably from a fermentation process. In contrast to the current methods prior art processes such as the extraction of oils from soybeans in which the soybeans must be dried, the process of the present invention does not require a drying step prior to the extraction process. Thus, processes of the present invention are applicable to extracting lipids from a microbial biomass containing at least about 10% by weight entrained water, preferably at least about 20%, more preferably at least about 30%, and most preferably at least about 50%. When the microorganisms are obtained from a fermentation process, the process of the present invention can include adding a base to the fermentation broth to dissolve any proteinaceous compound that may be present in the broth. "Bases" are substances which show alkali (basic) reactions in watery solutions, i.e. they attach protons and dissociate hydroxide ions. The base should be strong enough to hydrolyze or solubilize at least a portion of proteinaceous compounds that may be present in the broth. Bases which are useful for solubilizing proteins are well known to one of ordinary skill in the art of chemistry. Exemplary bases which are useful in the processes of the present invention include, but are not limited to, hydroxides, carbonates and bicarbonates of lithium, sodium, potassium, calcium, and magnesium carbonate. Other very basic compounds like basic phosphate salts, such as trisodium phosphate, can also be used.

The process of the present invention can also include rupturing or lysing the cells of microorganisms to release the lipids which are present within the cells. Cells can be lysed using any of the known methods including chemical; thermal; mechanical, including, but not limited to, french press, mills, ultrasonication, homogenization, and steam explosion; and combinations thereof. In a thermal lysing of cells, the fermentation broth containing microorganisms are heated until cells, *i.e.,* cell walls, of microorganisms degrade or breakdown. Typically, the fermentation broth is heated to a temperature of at least about 50°C, preferably at least about 75°C, more preferably to at least about 100°C, and most preferably to at least about 130°C. An important aspect of the process is to maintain the temperature below that temperature at which the extracted lipids can be degraded. Thermally lysing the cell walls of microorganisms is particularly useful for microorganisms whose cell walls are composed of proteins. During this process the headspace of the fermentor can be filled with nitrogen or another inert gas to prevent oxidation of the lipids by oxygen.

Heating the broth also denatures proteins and helps solubilize organic materials, including proteins. Heating of the fermentation broth step can be achieved by any of the known methods, including the use of an in-line heat exchanger, and preferably by sparging steam into the fermentation broth and maintaining the broth at a desired temperature for less than about 90 minutes, preferably less than about 60 minutes, and more preferably less than about 30 minutes.

The solventless extraction process of the present invention can also include at least partially separating the spent fermentation broth from the lipids. Typically, this is achieved by centrifuging, *e.g*., by passing the broth through a stacked-disc, separator or decantor centrifuge, and collecting lipids as an emulsion phase. Centrifuging the mixture results in a two phase mixture comprising a heavy layer and a light layer. Typically, the heavy layer is an aqueous phase, which contains the majority of cellular debris. The light layer which contains emulsified lipids is then diluted with water, again separated into two phase mixture and the light layer is again isolated. This dilution with water, separation and isolation processes (*i.e*., washing process) can be achieved continuously by feeding water and removing the heavy layer throughout the process or it can be conducted in discreet steps. The washing process is generally repeated until a substantially non-emulsified lipid layer is obtained, although minor amounts of emulsion can remain. It is believed that the oil-water interface of the emulsion is stabilized by residual cellular debris which is removed by the washing process. During the washing process, the successive amount of water added is reduced to increase the lipid content. While reducing the amount of feed water too quickly can result in loss of lipids to the aqueous phase, reducing the amount of feed water too slowly results in an inefficient washing process. One can readily determine an appropriate rate of feed water reduction by observing or analyzing the separated aqueous layer. Generally, the lipid layer, *i.e*., the light layer, is colored; therefore, in many cases one can determine an appropriate rate of feed water reduction by simply analyzing or observing the color of the aqueous layer which is separated from the lipid layer.

Alternatively, and preferably, the emulsion can be broken, and the oil recovered using the deoiling process outlined in WO 96/05278 which is incorporated herein by reference in its entirety. In this process a water soluble compound, e.g., alcohol and/or acetone, is added to the oil/water emulsion to break the emulsion and the resulting mixture is separated by centrifugation The isolated lipid can be further refined using a process similar to that used to refine standard vegetable oils. Briefly, the lipid refining process generally involves hydrating phospholipids by adding phosphoric acid to the lipid followed by adding sodiumium hydroxide to neutralize free fatty acids. These compounds are removed via centrifugation. This is then followed by a water wash step to further remove any remaining amounts of hydrated phospholipids ("gums") and neutralized fatty acids ("soapstock") in the lipid. The resulting lipid is bleached using Trysil™ and a standard bleaching clay. Citric acid is also added to remove divalent metal ions by chelation. The Trysil™ and bleaching clay are then removed via filtration to produce refined lipid. The bleached lipid can be cold filtered to remove high melting point compounds that may be present in the lipid; however, this step is generally seldom required.

The resulting lipid can be further refined by removing any low molecular weight components that may be present. Typically, these components are removed by sparging with steam at high temperatures, under high vacuum. This process also destroys any peroxide bonds which may be present and reduces or removes off- odors and helps improve the stability of the oil. An antioxidant may then be added to the resulting deodorized lipid to improve product stability.

During the refining process, the isolated lipid can be winterized to remove high melting compounds, such as saturated fatty acids. The winterization process generally involves dissolving the isolated lipid in an organic solvent, *e.g*., hexane, cooling the resulting organic solution, and filtering the solution to remove the high melting point components of the lipid or stearine phase. The winterization process generally produces a clear lipid, especially when the isolated lipid is cloudy or opaque. As will be appreciated, the use of a solvent such as hexane is acceptable in processes such as the above described "refining" process. Alternatively, the isolated lipid can be chilled and solidified impurities can be filtered out, without employing a solvent.

The refining, bleaching, deodorizing process outlined above would be used for triglyceride-rich lipid mixtures. Alternatively, or in addition to this process, other lipids, for example, pigments or carotenoids can be separated and purified, e.g., by partition into various solvents, chromatographic methods, etc.

While, the process of the present invention can include isolating microorganisms from a fermentation process, one of the advantages of the present invention is that it allows fermentation of microorganisms and isolation of lipids to be carried out in a single vessel. For example, after the fermentation, one can add base to the fermentation vessel and heat the mixture to lyse cells. After separating the phase into a heavy layer and a light layer, the light layer can be transferred to another vessel for further processing or the heavy layer can be removed from the fermentation vessel, for example, by draining through the bottom of the fermentation vessel, and the remaining light layer can be further processed within the same fermentation vessel.

If the concentration of lipids in the cells of the microbial culture is high (e.g., greater than about 20%) but the concentration of cells is low (e.g., less than about 40 g/L), as in cells grown in continuous fermentation systems, or cultures of difficult (e.g., fragile) to grow cells, or cultures produced in photosynthetically-based culture systems the cells can be concentrated, e.g., by centrifugation, filtration, or settling, prior to employing the methods of the invention if necessary.

Additional objects, advantages, and novel features of this invention will become apparent to those skilled in the art upon examination of the following examples thereof, which are not intended to be limiting.

### Examples

Process reproducibility was characterized by producing three samples of fully refined oil using crude oil from the new solventless extraction process. A hexane-extracted sample was also fully refined to serve as a control. The fermentation, extraction and oil isolation steps were performed at a large scale, while the oil refining studies were performed at a small scale.

The fully refined oil samples were analyzed to demonstrate process reproducibility.

### Fermentation:

An oil-rich microorganism *(Schizochytrium sp.)* was grown in a 1200 gallon fermenter to produce a fermentation broth for the following extraction processes. A single batch was used to generate the starting broth for the three solventless extraction processes. The fermentation was allowed to run for 94 hours, while controlling the glucose levels at 13 g/L, after which time the corn syrup feed was terminated. Residual glucose levels dropped to <5 g/L four hours later. This resulted in a final age of 98 hours. The final broth volume was 958 gallons. The final yield was 146 g/L dry weight of cells. Both in-process contamination checks and a thorough analysis of a final broth sample failed to show any signs of contamination.

### Hexane-Extracted Control Sample:

A small aliquot of broth from the fermentation batch was drum-dried and extracted with hexane to serve as a control sample. The biomass intermediate was recovered using a double-drum dryer. Analysis of this lipid is shown in Table 1.

**Table 1. Analysis of drum dried Schizochytrium sp. biomass.**

| Parameter | Value |
|---|---|
| DHA Content (FAME basis) | 35.7% |
| Oil Content | 62.7% |
| Peroxide Value (meq/kg) | 2.6 |
| Total Plate Count (cfu/g) | <50 |
| DHA Content^{*} | 20.3% |
| FAME Content | 56.9% |

| | |
|---|---|
| * cellular dry weight basis | |

### Solventless Extraction Process:

Crude oil was obtained by treating three 400-gallon aliquots (approx.) of the fermentation broth. Each 400-gallon aliquot from the fermentor was processed separately, starting with the caustic/heat treatment steps. Each aliquot was treated with 20 grams of 45% KOH per liter and heated to 130°C for about 30 minutes by passing steam through the fermentation broth. The crude oil was recovered from the treated broth using a commercial-scale Westfalia HFA-100 stacked-disc centrifuge. Summary results for various process parameters are reported in Table 2, and the final crude oil analysis results are shown in Table 3.

**Table 2. Process Data from the Solventless Extraction Process.**

| | | SFE-1 | SFE-2 | SFE-3 |
|---|---|---|---|---|
| **Broth Treatment** | | | | |
| | Volume of Broth Processed | 288 gal | 288 gal | 258 gal |
| | Final Treated pH | 7.5 | 8.0 | 8.7 |
| | Final Volume After Heat Treatment | 388 gal | 398 gal | 308 gal |
| | Volume Increase From Condensate | 34.7% | 38.2% | 19.4% |

| **1^{st} Pass Emulsion** | | | | |
|---|---|---|---|---|
| | Total Volume (gal) | 180 | 133 | 149 |
| | Est. Oil Concentration (w/w) | 12.0% | 24.5% | 16.1% |
| | Apparent Density (g/mL) | 0.986 | 0.991 | 0.999 |

| **Oil Isolation** | | | | |
|---|---|---|---|---|
| | Total Crude Oil Recovered (1b) | 182 | 165 | 174 |
| | | | | |
| | DHA Oil Lot Number Assigned | SF1A | SF2A | SF3A |

**Table 3. Analysis of Lots of DHA oil from the Solventless Extraction Process.**

| Parameter | SF1A | SF2A | SF3A |
|---|---|---|---|
| DHA Content (%FAME) | 39.0% | 38.6% | 39.2% |
| Peroxide Value (meq/kg) | 4.6 | 1.8 | 2.0 |
| Acid Value (mg KOH/g) | N/D | N/D | N/D |
| Moisture Content | N/D | N/D | N/D |

### Refining:

A sample from each aliquot of crude oil was winterized, refined, bleached and deodorized at a small scale, as was a sample of the crude oil from the hexane-extracted control. Miscellaneous process data from these small scale experiments is shown in Table 4, including recovery efficiencies for the various processing steps. While it is difficult to read too much into recovery efficiencies for bench-scale processes, as losses tend to be disproportionately large, the values listed in Table 4 show that values for the solventless-extracted samples tend to bracket the values measured for the hexane-extracted control, with the one exception being the winterization step. While the recovery efficiency during the winterization step for the hexane control was lower than those observed for the other three samples, this difference is insignificant from a statistical perspective. The high losses during the winterization step caused the overall recovery efficiency for the hexane-control sample to be lower as well. The lower yield would not be expected to have a significant impact on the overall quality of the oil. All in all, differences in the processing of the various oil samples were minimal.

**Table 4. Miscellaneous Process Data from the Oil Refining Steps.**

| | | HEX-1 | SF1A | SF2A | SF3A |
|---|---|---|---|---|---|
| **Processing Conditions** | | | | | |
| | Miscella Concentration | 45.0% | 52.9% | 52.8% | 45.0% |
| | Steam Sparge Rate | 3.4% | 3.4% | 2.5% | 2.2% |

| **Recovery Efficiencies** | | | | | |
|---|---|---|---|---|---|
| | Winterization | 80.6% | 92.3% | 87.7% | 85.5% |
| | Refining | 89.4% | 84.8% | 91.8% | 95.0% |
| | Water Wash | 90.6% | 94.5% | 95.8% | 81.2% |
| | Bleaching | 86.1% | 89.2% | 87.3% | 84.1% |
| | Deodorization | 97.4% | 96.1% | 97.2% | 97.5% |
| | Packaging | 88.2% | 89.7% | 89.3% | 95.8% |
| | **Overall** | **48.2%** | **56.9%** | **58.5%** | **51.8%** |

Fully refined oil samples from the three solventless extraction runs, and the hexane-extracted control, were analyzed and the results are shown in Table 5. Also shown are the corresponding release specifications for each parameter.

A sample of the starting crude oil from the solventless extraction run was also analyzed for iron content. The iron content of this sample was 0.08 ppm. The concentration of the other trace metals was all below their respective detection limits.

**Table 5. QC Results for RBD DHA Oil from the Solventless Extraction Process compared to hexane extracted oil.**

| Hexane | | | Solventless Extrac. Process | | |
|---|---|---|---|---|---|
| Run ID # | | HEX-1 | SFA1 | SFA2 | SFA3 |
| | | | | | |
| Peroxide Value (meq/kg) | | 0.28 | 0.69 | 0.35 | 0.34 |
| Acid Value (mg KOH/g) | | 0.17 | 0.11 | 0.57 | 0.24 |
| Moisture & Volatiles | | 0.00% | 0.06%** | 0.00% | 0.00% |

| Trace Metals (ppm) | | | | | |
|---|---|---|---|---|---|
| | Lead | <0.20 | <0.20 | <0.20 | <0.20 |
| | Arsenic | <0.20 | <0.20 | <0.20 | <0.20 |
| | Iron | 0.22 | 0.21 | 0.56*** | 0.02 |
| | Copper | <0.05 | <0.05 | <0.05 | <0.05 |
| | Mercury | <0.20 | <0.20 | <0.20 | <0.20 |
| DHA (%FAME) | | 36.9 | 37.3 | 37.0 | 37.7 |
| DHA (mg/g oil) | | 342 | 345 | 343 | 351 |
| Hexane (ppm) | | <3 | <3 | <3 | <3 |

| | | | | | |
|---|---|---|---|---|---|
| * Value was reduced to 0.22 mg KOH/g after repeating the refining and bleaching steps ** Sample analyzed by the San Diego Fermentation Sciences Analytical Group. *** Value was reduced to <0.02 ppm after repeating the refining and bleaching steps | | | | | |

Shown in Table 6 is a more direct comparison of the average analysis results for the three samples from the solventless extraction process versus those for the hexane control.

**Table 6. Comparison of Average Values.**

| Hexane | | | Solventless Extraction | | | |
|---|---|---|---|---|---|---|
| Parameter | | Control | Mean | Std Dev | CV | %Diff |
| Peroxide Value (meq/kg) | | 0.28 | 0.46 | 0.20 | 43.3% | 64.3% |
| Acid Value (mg KOH/g) | | 0.17 | 0.19* | 0.06 | 33.3% | 11.2% |
| Moisture & Volatiles | | 0.00% | 0.02% | 0.03% | 173% | ND |
| Trace Metals (ppm) | | | | | | |
| | Lead | <0.20 | <0.20 | N/A | N/A | 0.0% |
| | Arsenic | <0.20 | <0.20 | N/A | N/A | 0.0% |
| | Iron | 0.22 | 0.26 | 0.27 | 104% | 18.2% |
| | Copper | <0.05 | <0.05 | N/A | N/A | 0.0% |
| | Mercury | <0.20 | <0.20 | N/A | N/A | 0.0% |
| DHA Content (%FAME) | | 36.9% | 37.3% | 0.4% | 0.9% | 1.1% |
| DHA Content (mg/g) | | 342 | 346 | 4 | 1.2% | 1.2% |
| Hexane (ppm) | | <3 | <3 | N/A | N/A | 0.0% |

| | | | | | | |
|---|---|---|---|---|---|---|
| * Calculated using the acid value for the re-worked sample. | | | | | | |

The results from this experiment clearly demonstrate that the solventless extraction process is both reproducible and lipids from solventless extraction are relatively indistinguishable from the lipids obtained from hexane extraction process in terms of process performance and product quality. The final product from the solventless extraction process is substantially equivalent to lipids from a current hexane-based extraction process, as determined by similarities between the fatty acid and sterol profiles of the product from these two processes.

The present invention, in various embodiments, includes components, methods, processes, systems and/or apparatus substantially as depicted and described herein, including various embodiments, subcombinations, and subsets thereof. Those of skill in the art will understand how to make and use the present invention after understanding the present disclosure. The present invention, in various embodiments, includes providing devices and processes in the absence of items not depicted and/or described herein or in various embodiments hereof, including in the absence of such items as may have been used in previous devices or processes, *e.g*., for improving performance, achieving ease and/or reducing cost of implementation.

The foregoing discussion of the invention has been presented for purposes of illustration and description. The foregoing is not intended to limit the invention to the form or forms disclosed herein. Although the description of the invention has included description of one or more embodiments and certain variations and modifications, other variations and modifications are within the scope of the invention, *e.g*., as may be within the skill and knowledge of those in the art, after understanding the present disclosure. It is intended to obtain rights which include alternative embodiments to the extent permitted, including alternate, interchangeable and/or equivalent structures, functions, ranges or steps to those claimed, whether or not such alternate, interchangeable and/or equivalent structures, functions, ranges or steps are disclosed herein, and without intending to publicly dedicate any patentable subject matter.

Various preferred features and embodiments of the present invention will now be described with reference to the following numbered paragraphs (paras).
1. A process for obtaining lipid from microorganisms comprising:
   (a) lysing cells of the microorganisms to produce a lysed cell mixture;
   (b) treating said lysed cell mixture to produce a phase separated mixture comprising a heavy layer and a light layer, wherein said heavy layer comprises an aqueous solution and said light layer comprises said lipid;
   (c) separating said heavy layer from said light layer; and
   (d) obtaining said lipid from said light layer.
2. The process of para 1, wherein said step (b) comprises centrifuging said lysed cell mixture.
3. The process of para 2, wherein said light layer comprises an emulsified lipid.
4. The process of para 3 further comprising:
   (e) adding an aqueous extraction solution to said light layer of step (c); and
   (f) repeating said steps (c), (d) and (e) until said lipid becomes substantially non-emulsified prior to said step (d).
5. The process of para 3, wherein said emulsified lipid comprises a suspension of said lipid in an aqueous solution.
6. The process of para 1, wherein said aqueous solution comprises solid cell materials.
7. The process of para 1, wherein said microorganisms are obtained from a fermentation process.
8. The process of para 7 further comprising adding a base to a fermentation broth.
9. The process of para 8, wherein said base is selected from the group consisting of hydroxides, carbonates, bicarbonates, phosphates and mixtures thereof.
10. The process of para 7 further comprising solubilizing at least part of proteinaceous compounds in a fermentation broth.
11. The process of para 1, wherein said step (a) comprises heating said microorganisms to temperature of at least about 50°C.
12. The process of para 1, wherein said microorganism is capable of growth at salinity level of less than about 12 g/L of sodium chloride.
13. The process of para 1, wherein said microorganism comprises at least about 20% by weight of lipid.
14. The process of para 1, wherein said microorganism is selected from the group consisting of algae, fungi, bacteria and protists.
15. The process of para 14, wherein said microorganisms comprise microorganisms of the order Thraustochytriales.
16. The process of para 15, wherein said microorganisms are selected from the genus *Thraustochytrium, Schizochytrium* and mixtures thereof.
17. The process of para 16, wherein said microorganisms are selected from the group consisting of microorganisms having the identifying characteristics of ATCC number 20888, ATCC number 20889, ATCC number 20890, ATCC number 20891 and ATCC number 20892, mutant strains derived from any of the foregoing, and mixtures thereof.
18. The process of para 1, wherein said microorganisms are capable of producing at least about 0.1 grams per liter per hour of cholesterol, phytosterols, desmosterol, tocotrienols, tocopherols, ubiquinones, carotenoids and xanthophylls such as beta-carotene, lutein, lycopene, astaxanthin, zeaxanthin, canthaxanthin, and fatty acids such as conjugated linoleic acids, and omega-3, and omega-6 highly unsaturated fatty acids such as eicosapentaenoic acid, docosapentaenoic acid, and docosahexaenoic acid, arachidonic acid, stearidonic acid, dihomogammalinolenic acid and gamma-linolenic acid or mixtures thereof.
19. The process of para 1, wherein at least about 20% of said lipid is cholesterol, phytosterols, desmosterol, tocotrienols, tocopherols, ubiquinones, carotenoids and xanthophylls such as beta-carotene, lutein, lycopene, astaxanthin, zeaxanthin, canthaxanthin, and fatty acids such as conjugated linoleic acids, and omega-3 and omega-6 highly unsaturated fatty acids such as eicosapentaenoic acid, docosapentaenoic acid, and docosahexaenoic acid, arachidonic acid, stearidonic acid, dihomogammalinolenic acid and gamma-linolenic acid or mixtures thereof.
20. A process for obtaining lipids from microorganisms comprising:
   (a) growing said microorganisms in a fermentation medium to produce a fermentation broth;
   (b) solubilizing at least a part of any proteins present in said fermentation broth;
   (c) lysing cells of said microorganisms to produce a lysed cell mixture;
   (d) treating said lysed cell mixture to produce a phase separated mixture comprising a heavy layer and a light layer, wherein said heavy layer comprises an aqueous solution and said light layer comprises emulsified lipids;
   (e) separating said heavy layer from said light layer; and
   (f) obtaining said lipids from said light layer.
21. The process of para 20, wherein said step of dissolving proteins comprises contacting said fermentation broth with a base.
22. The process of para 21, wherein said base is selected from the group consisting of hydroxides, carbonate, bicarbonates, phosphates and mixtures thereof.
23. The process of para 20, wherein said step of lysing cells comprises heating said microorganisms to a temperature of at least about 50°C.
24. The process of para 20, wherein said step of producing the phase separated mixture comprises centrifuging said lysed cell mixture.
25. The process of para 20, wherein said step of obtaining said lipids from said light layer comprises:
   (a) adding an aqueous washing solution to said light layer;
   (b) separating said aqueous washing solution from said light layer; and
   (c) repeating said steps (A) and (B) until said lipid becomes substantially non-emulsified.
26. The process of para 20, wherein said aqueous solution comprises solid cell materials.
27. The process of para 20, wherein said microorganism is capable of growth at salinity level of less than about 12 g/L of sodium chloride.
28. The process of para 20, wherein said microorganism comprises at least about 20% by weight of lipid.
29. The process of para 20, wherein said microorganism is selected from the group consisting of algae, fungi, bacteria and protists.
30. The process of para 29, wherein said microorganisms comprise microorganisms of the order Thraustochytriales.
31. The process of para 30, wherein said microorganisms are selected from the genus *Thraustochytrium, Schizochytrium* and mixtures thereof.
32. The process of para 31, wherein said microorganisms are selected from the group consisting of microorganisms having the identifying characteristics of ATCC number 20888, ATCC number 20889, ATCC number 20890, ATCC number 20891 and ATCC number 20892, mutant strains derived from any of the foregoing, and mixtures thereof.
33. The process of para 20, wherein said microorganisms are capable of producing at least about 0.1 grams per liter per hour of cholesterol, phytosterols, desmosterol, tocotrienols, tocopherols, ubiquinones, carotenoids and xanthophylls such as beta-carotene, lutein, lycopene, astaxanthin, zeaxanthin, canthaxanthin, and fatty acids such as conjugated linoleic acids, and omega-3 and omega-6 highly unsaturated fatty acids such as eicosapentaenoic acid, docosapentaenoic acid, and docosahexaenoic acid, arachidonic acid, stearidonic acid, dihomogammalinolenic acid and gamma-linolenic acid or mixtures thereof.
34. The process of para 20, wherein at least about 20% of said lipid is cholesterol, phytosterols, desmosterol, tocotrienols, tocopherols, ubiquinones, carotenoids and xanthophylls such as beta-carotene, lutein, lycopene, astaxanthin, zeaxanthin, canthaxanthin, and fatty acids such as conjugated linoleic acids, and omega-3 and omega-6 highly unsaturated fatty acids such as eicosapentaenoic acid, docosapentaenoic acid, and docosahexaenoic acid, arachidonic acid, stearidonic acid, dihomogammalinolenic acid and gamma-linolenic acid or mixtures thereof.
35. A process for obtaining lipids from microorganisms comprising:
   (a) growing said microorganisms in a fermentation medium to produce a fermentation broth;
   (b) contacting said fermentation broth with a base to dissolve at least a part of any proteins present in said fermentation broth;
   (c) increasing the temperature of said fermentation broth to at least about 50°C to lyse cells of said microorganisms to produce a lysed cell mixture;
   (d) separating substances of different densities from said lysed cell mixture to produce a phase separated mixture comprising a heavy layer and a light layer, wherein said heavy layer comprises an aqueous solution and said light layer comprises emulsified lipids;
   (e) removing said heavy layer from said phase separated mixture;
   (f) adding an aqueous washing solution to said light layer;
   (g) separating substances of different densities from said mixture of step (f);
   (h) removing said heavy layer from said phase separated mixture; and
   (i) repeating said steps (f)-(h) until said lipid becomes substantially non-emulsified.
36. The process of para 35, wherein said base is selected from the group consisting of hydroxides, carbonates, bicarbonates, and mixtures thereof.
37. The process of para 35, wherein said step of producing the phase separated mixture comprises centrifuging said lysed cell mixture.
38. The process of para 35, wherein said aqueous solution of step (d) comprises solid cell materials.
39. The process of para 35, wherein said microorganism is capable of growth at salinity level of less than about 12 g/L of sodium chloride.
40. The process of para 35, wherein said microorganism comprises at least about 20% by weight of lipid.
41. The process of para 35, wherein said microorganism is selected from the group consisting of algae, fungi, bacteria and protists.
42. The process of para 35, wherein said microorganisms comprise microorganisms from the group consisting of golden algae, green algae, dinoflagelates, yeast, fungi of the genus *Mortierella,* and Stramenopiles.
43. The process of para 35, wherein said microorganisms comprise microorganisms of the order Thraustochytriales.
44. The process of para 43, wherein said microorganisms are selected from the group consisting of microorganisms having the identifying characteristics of ATCC number 20888, ATCC number 20889, ATCC number 20890, ATCC number 20891 and ATCC number 20892, mutant strains derived from any of the foregoing, and mixtures thereof.
45. The process of para 35, wherein said microorganisms are capable of producing at least about 0.1 grams per liter per hour of cholesterol, phytosterols, desmosterol, tocotrienols, tocopherols, ubiquinones, carotenoids and xanthophylls such as beta-carotene, lutein, lycopene, astaxanthin, zeaxanthin, canthaxanthin, and fatty acids such as conjugated linoleic acids, and omega-3 and omega-6 highly unsaturated fatty acids such as eicosapentaenoic acid, docosapentaenoic acid, and docosahexaenoic acid, arachidonic acid, stearidonic acid, dihomogammalinolenic acid and gamma-linolenic acid or mixtures thereof.
46. The process of para 35, wherein at least about 20%of said lipid is cholesterol, phytosterols, desmosterol, tocotrienols, tocopherols, ubiquinones, carotenoids and xanthophylls such as beta-carotene, lutein, lycopene, astaxanthin, zeaxanthin, canthaxanthin, and fatty acids such as conjugated linoleic acids, and omega-3 and omega-6 highly unsaturated fatty acids such as eicosapentaenoic acid, docosapentaenoic acid, and docosahexaenoic acid, arachidonic acid, stearidonic acid, dihomogammalinolenic acid and gamma-linolenic acid or mixtures thereof.
47. A process for obtaining lipids for microorganisms comprising:
   (a) growing microorganisms in a culture medium;
   (b) treating said culture medium and the microorganism cells to release intercellular lipids;
   (c) subjecting the culture medium containing the released intercellular lipids to gravity separation to form a light lipid-containing phase and a heavy phase;
   (d) separating said light phase from said heavy phase;
   (e) treating said light phase to break an emulsion formed between said lipid and water; and
   (f) recovering a crude lipid.
48. The process of para 47 wherein said microorganisms are lipid-rich microorganisms.
49. The process of para 47 wherein said microorganisms are selected from the group consisting of algae, bacteria, fungi and protists.
50. The process of para 47 wherein said microorganisms are selected from the group consisting of golden algae, green algae, dinoflagelates, yeast, fungi of the genus *Mortierella,* and Stramenopiles.
51. The process of para 47, wherein said microorganisms comprise microorganisms of the order Thraustochytriales.
52. The process of para 51, wherein said microorganisms are selected from the genus *Thraustochytrium, Schizochytrium* and mixtures thereof.
53. The process of para 52, wherein said microorganisms are selected from the group consisting of microorganisms having the identifying characteristics of ATCC number 20888, ATCC number 20889, ATCC number 20890, ATCC number 20891 and ATCC number 20892, mutant strains derived from any of the foregoing, and mixtures thereof.
54. The process of para 47, wherein said microorganisms are capable of producing at least about 0.1 grams per liter per hour of cholesterol, phytosterols, desmosterol, tocotrienols, tocopherols, ubiquinones, carotenoids and xanthophylls such as beta-carotene, lutein, lycopene, astaxanthin, zeaxanthin, canthaxanthin, and fatty acids such as conjugated linoleic acids, and omega-3 and omega-6 highly unsaturated fatty acids such as eicosapentaenoic acid, docosapentaenoic acid, and docosahexaenoic acid, arachidonic acid, stearidonic acid, dihomogammalinolenic acid and gamma-linolenic acid or mixtures thereof.
55. The process of para 54, wherein at least about 20% of said lipid is cholesterol, phytosterols, desmosterol, tocotrienols, tocopherols, ubiquinones, carotenoids and xanthophylls such as beta-carotene, lutein, lycopene, astaxanthin, zeaxanthin, canthaxanthin, and fatty acids such as conjugated linoleic acids, and omega-3 and omega-6 highly unsaturated fatty acids such as eicosapentaenoic acid, docosapentaenoic acid, and docosahexaenoic acid, arachidonic acid, stearidonic acid, dihomogammalinolenic acid and gamma-linolenic acid or mixtures thereof.
56. The process of para 47, wherein said treatment includes a treatment selected from the group consisting of heating said cells, exposing said cells to a basic compound, exposing said cells to a chelating compound or combinations thereof.
57. The process of para 47, wherein said treatment comprises heating the cells to at least 50°C before, during or after exposing the cells to a basic compound, a chelating compound or mixtures thereof.
58. The process of para 47, wherein said gravity separation of step (c) comprises passing the culture medium containing the released intercellular lipids through a stacked-disc, separator or decantor centrifuge.
59. The process of para 47, wherein the treatment of step to break the emulsion comprises mixing the emulsion with water, alcohol and/or acetone and subjecting the mixture to gravity separation.
60. The process of para 59, wherein said gravity separation comprises centrifugation.
61. The process of para 60, wherein said centrifugation includes treatment in a stacked-disc-, separator- or decanter-type centrifuge.
62. The process of para 59, wherein said treatment is repeated at least 3 times to obtain said crude lipid.
63. The process of para 47, wherein said crude lipid is subjected to further refining or processing to obtain a refined lipid.
64. The process of para 63, wherein said crude lipid is bleached and deodorized.
65. A process for recovering lipids from microorganisms comprising the steps:
   a. growing said microorganisms in a culture medium;
   b. treating microorganism cells from said culture medium without drying said cells to release intercellular lipids;
   c. subjecting the culture medium containing the released intercellular lipids to gravity separation to form a light lipid-containing phase and a heavy phase;
   d. separating said light phase from said heavy phase;
   e. treating said light phase to break an emulsion formed between said lipid and water; and
   f. recovering a crude lipid.
66. The process of para 65 wherein said microorganisms are lipid-rich microorganisms.
67. The process of para 65 wherein said microorganisms are selected from the group consisting of algae, bacteria, fungi and protists.
68. The process of para 65 wherein said microorganisms are selected from the group consisting of golden algae, green algae, dinoflagelates, yeast, fungi of the genus *Mortierella,* and Stramenopiles.
69. The process of para 65, wherein said microorganisms comprise microorganisms of the order Thraustochytriales.
70. The process of para 69, wherein said microorganisms are selected from the genus *Thraustochytrium, Schizochytrium* and mixtures thereof.
71. The process of para 70, wherein said microorganisms are selected from the group consisting of microorganisms having the identifying characteristics of ATCC number 20888, ATCC number 20889, ATCC number 20890, ATCC number 20891 and ATCC number 20892, mutant strains derived from any of the foregoing, and mixtures thereof.
72. The process of para 65, wherein said microorganisms are capable of producing at least about 0.1 grams per liter per hour of cholesterol, phytosterols, desmosterol, tocotrienols, tocopherols, ubiquinones, carotenoids and xanthophylls such as beta-carotene, lutein, lycopene, astaxanthin, zeaxanthin, canthaxanthin, and fatty acids such as conjugated linoleic acids, and omega-3 and omega-6 highly unsaturated fatty acids such as eicosapentaenoic acid, docosapentaenoic acid, and docosahexaenoic acid, arachidonic acid, stearidonic acid, dihomogammalinolenic acid and gamma-linolenic acid or mixtures thereof.
73. The process of para 72, wherein at least about 20% of said lipid is cholesterol, phytosterols, desmosterol, tocotrienols, tocopherols, ubiquinones, carotenoids and xanthophylls such as beta-carotene, lutein, lycopene, astaxanthin, zeaxanthin, canthaxanthin, and fatty acids such as conjugated linoleic acids, and omega-3 and omega-6 highly unsaturated fatty acids such as eicosapentaenoic acid, docosapentaenoic acid, and docosahexaenoic acid, arachidonic acid, stearidonic acid, dihomogammalinolenic acid and gamma-linolenic acid or mixtures thereof.
74. The process of para 65, wherein said treatment of the cells includes a treatment selected from the group consisting of heating said cells, exposing said cells to basic conditions, exposing said cells to a chelating compound or combinations thereof.
75. The process of para 65, wherein said treatment of the cells comprises heating the cells to at least 50°C before, during or after exposing the cells to a basic condition, a chelating compound or mixtures thereof.
76. The process of para 65, wherein said gravity separation of step (c) comprises passing the culture medium containing the released intercellular lipids through a stacked-disc, separator or decantor centrifuge.
77. The process of para 65, wherein the treatment to break the emulsion comprises mixing the emulsion with water, alcohol and/or acetone and subjecting the mixture to gravity separation.
78. The process of para 77, wherein said gravity separation comprises centrifugation.
79. The process of para 78, wherein said centrifugation includes treatment in a stacked-disc-, separator- or decanter-type centrifuge.
80. The process of para 77, wherein said treatment is repeated at least 3 times to obtain said crude lipid.
81. The process of para 65, wherein said crude lipid is subjected to further refining or purification to obtain a refined lipid.
82. The process of para 81, wherein said crude lipid is bleached and deodorized.
83. A lipid made by the process of paras 1-82.
84. A lipid derived from microorganisms with less than 0.2ppm residual nonpolar organic solvent.
85. A lipid comprising greater than 15% cholesterol, phytosterols, desmosterol, tocotrienols, tocopherols, ubiquinones, carotenoids and xanthophylls such as beta-carotene, lutein, lycopene, astaxanthin, zeaxanthin, canthaxanthin, and fatty acids such as conjugated linoleic acids, and omega-3 and omega-6 highly unsaturated fatty acids such as eicosapentaenoic acid, docosapentaenoic acid, and docosahexaenoic acid, arachidonic acid, stearidonic acid, dihomogamnalinolenic acid and gamma-linolenic acid or mixtures thereof with less than 0.2 ppm residual nonpolar organic solvent.A lipid comprising greater than 15% docosahexaenoic acid and less than 0.2 ppm residual nonpolar organic solvent.

## Claims

1. A process for obtaining lipid from a microorganism, comprising:
(a) lysing cells of the microorganism to produce a lysed cell mixture;
(b) treating the lysed cell mixture using an extraction process conducted in a medium that comprises less than 5% nonpolar organic solvent to produce a phase separated mixture comprising a heavy layer and a light layer, wherein the heavy layer comprises an aqueous solution and the light layer comprises the lipid;
(c) separating the heavy layer from the light layer; and
(d) obtaining the lipid from the light layer.

2. The process of claim 1, wherein (b) comprises centrifuging the lysed cell mixture.

3. The process of claim 1 or 2, wherein the light layer comprises an emulsified lipid.

4. The process of claim 3, further comprising:
(e) adding an aqueous extraction solution to the light layer of (c); and
(f) repeating (b), (c) and (e) until the lipid becomes substantially non-emulsified prior to (d).

5. The process of claim 3, wherein the emulsified lipid comprises a suspension of the lipid in an aqueous solution.

6. The process of any of claims 1-5, further comprising adding a base to the fermentation medium.

7. The process of claim 6, wherein the base is selected from the group consisting of hydroxides, carbonates, bicarbonates, and mixtures thereof.

8. The process of any of claims 1-7, further comprising solubilizing at least part of proteinaceous compounds in the fermentation medium.

9. The process of any of claims 1-8, wherein (a) comprises heating the microorganism to a temperature of at least 50 °C.

10. The process of any of claims 1-9, wherein the microorganism is capable of growth at a salinity level of less than 12 g/L of sodium chloride.

11. The process of any of claims 1-10, wherein the microorganism comprises at least 30% by weight of lipid.

12. The process of any of claims 1-11, wherein the microorganism is selected from the group consisting of algae, fungi, bacteria and protist.

13. The process of any of claims 1-12, wherein the microorganism comprises a microorganism of the order Thraustochytriales.

14. The process of any of claims 1-13, wherein the microorganism is selected from the genus *Thraustochytrium, Schizochytrium* and mixtures thereof.

15. The process of any of claims 1-14, wherein the microorganism is selected from the group consisting of microorganisms having the identifying characteristics of ATCC number 20888, ATCC number 20889, ATCC number 20890, ATCC number 20891, ATCC number 20892, and mutant strains derived from any of the foregoing.

16. The process of any of claims 1-15, wherein the microorganism is capable of producing at least 0.1 grams per liter per hour of docosahexaenoic acid.

17. The process of any of claims 16, wherein at least 30 % of said lipid is docosahexaenoic acid.

18. The process of any of claims 1-17, wherein (b) is conducted in less than 4%, less than 2%, or less than 1% of a nonpolar organic solvent.

19. The process of claim 18, wherein the nonpolar organic solvent is hexane.

20. The process of any of claims 1-17, wherein (b) is conducted in the absence of a nonpolar organic solvent.
